# EUROPEAN PATENT APPLICATION

(11) **EP 3 974 888 A1**
(43) Date of publication of application: **30.03.2022**
(21) Application number: 20832543.1
(22) Date of filing: 27.05.2020
(51) Int. Cl.: G02B 23/26, A61B 1/06

(54) **MEDICAL LIGHTING DEVICE**

(30) Priority: 26.06.2019 JP 2019118618
(71) Applicant: Sony Olympus Medical Solutions Inc., Hachioji-shi, Tokyo 192-0904 (JP)
(72) Inventor: KITAMURA, Ken, Hachioji-shi, Tokyo 192-0904 (JP)
(74) Representative: D Young & Co LLP
(86) International application number: PCT/JP2020/020994
(87) International publication number: WO 2020/261857

(57) **Abstract**

A medical lighting device according to the present invention includes a light source unit that emits light, an optical fiber that guides and emits the light having emitted from the light source unit and having entered inside the optical fiber, first and second detection units that are provided in the vicinity of an emission end including an emission end for the light of the optical fiber and detect the light in the optical fiber, and a control unit that determines, based on detection values of the light detected by the first and second detection units, normality/abnormality in the detection values of the respective detection units and outputs determined results.

## Description

### Field

The present invention relates to a medical lighting device.

### Background

Conventionally in the medical fields and industrial fields, medical devices, such as endoscope apparatuses and medical microscope devices, that capture object images by using image sensors, are known (e.g., see Patent Literature 1). Among the medical devices, the endoscope apparatus includes, for example, an endoscope, an imaging device, a display device, a control device (image processing device), and a light source device. In the endoscope apparatus, illumination light that is supplied from the light source device via a light guide connected to the endoscope is emitted to capture an object image.

A light source device includes a light source and an optical fiber that guides light from the light source. In Patent Literature 1, an optical sensor that detects an amount of light guided by an optical fiber is provided to control an amount of light emitted from the light source device. The optical sensor receives light leaked from the optical fiber and detects an amount of light leaked.

### Citation List

### Patent Literature

Patent Literature 1: JP 2015-210890 A

### Summary

### Technical Problem

In Patent Literature 1, one detection unit is provided for the optical fiber. At this time, when a detection value of the amount of light has an abnormal value different from that in a normal state, it is impossible to determine whether the abnormal value indicates a failure in the optical sensor or the abnormal value indicates an abnormality in the optical fiber. For this reason, it is impossible to identify which of the optical fiber and the optical sensor has an abnormal portion.

The present invention has been made in view of the above, and an object of the present invention is to provide a medical lighting device that is configured to identify which of an optical fiber and an optical sensor has an abnormal portion.

### Solution to Problem

To solve the above-described problem and achieve the object, a medical lighting device according to the present invention includes: an emission unit configured to emit light; an optical fiber configured to guide and emit the light emitted from the emission unit and entered into the optical fiber; first and second detection units provided in a vicinity of an emission end including an emission end for the light of the optical fiber and configured to detect the light in the optical fiber; and a control unit configured to determine, based on detection values of the light detected by the first and second detection units, normality/abnormality in the detection values of the respective detection units and outputs determined results.

Moreover, in the medical lighting device according to the present invention, the control unit is configured to determine, based on results of the determination of the normality/abnormality in the detection values of the respective detection units, normality/abnormality of the first and second detection units and the optical fiber.

Moreover, in the medical lighting device according to the present invention, the control unit is configured to control to stop emission of the light by the emission unit in a case where at least one of the first and second detection units and the optical fiber is determined to be abnormal.

Moreover, in the medical lighting device according to the present invention, the control unit is configured to determine that the second detection unit is abnormal in a case where the detection value of the first detection unit is normal and the detection value of the second detection unit is abnormal.

Moreover, in the medical lighting device according to the present invention, the control unit is configured to determine that the optical fiber is abnormal in a case where the detection value of the first detection unit and the detection value of the second detection unit are abnormal.

Moreover, in the medical lighting device according to the present invention, the light emitted from the emission unit is excitation light for exciting an irradiation target.

Moreover, in the medical lighting device according to the present invention, a current monitoring unit configured to monitor current supplied to the emission unit is further included, and the control unit is configured to determine normality/abnormality of the detection unit, and determine normality/abnormality of the current monitoring unit.

Moreover, in the medical lighting device according to the present invention, the optical fiber includes a single fiber.

### Advantageous Effects of Invention

According to the present invention, it is possible to effectively identify which of the optical fiber and the optical sensor has an abnormal portion.

### Brief Description of Drawings

FIG. 1 is a diagram illustrating a schematic configuration of an endoscope apparatus according to a first embodiment of the present invention.
FIG. 2 is a block diagram illustrating configurations of a camera head, a control device, and a light source device that are illustrated in FIG. 1.
FIG. 3 is a diagram illustrating a configuration of a main portion of the light source device illustrated in FIG. 1.
FIG. 4 is a diagram illustrating determination patterns for determining a normal/abnormal state in a light source unit, by a light source controller.
FIG. 5 is a flowchart illustrating a process of determining the normal/abnormal state in a first light source unit by the light source controller according to the first embodiment of the present invention.
FIG. 6 is a diagram illustrating a configuration of a main portion of a light source device in an endoscope apparatus according to a second embodiment of the present invention.
FIG. 7 is a flowchart illustrating a process of determining a normal/abnormal state in a first light source unit by a light source controller according to the second embodiment of the present invention.
FIG. 8 is a diagram schematically illustrating an overall configuration of a surgical microscope system being a medical observation system including a medical lighting device according to another embodiment.

### Description of Embodiments

Hereinafter, modes for implementing the present invention (hereinafter referred to as "embodiments") will be described. In the embodiments, as an example of a system including a medical lighting device according to the present invention, a medical endoscope apparatus that captures and displays an in-vivo image of a subject such as a patient will be described. Furthermore, the present invention is not limited to the embodiments. Still furthermore, in the drawings, the same portions are denoted by the same reference numerals, for description.

### (First Embodiment)

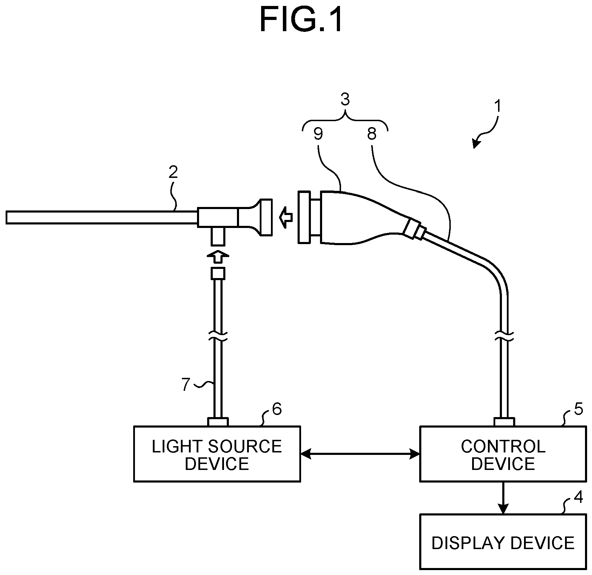

FIG. 1 is a diagram illustrating a schematic configuration of an endoscope apparatus 1 according to a first embodiment of the present invention. The endoscope apparatus 1 is an apparatus that is used in the medical field to observe an object inside an observation target (inside a living body), such as a human. As illustrated in FIG. 1, the endoscope apparatus 1 includes an endoscope 2, an imaging device 3, a display device 4, a control device 5, and a light source device 6, and the imaging device 3 and the control device 5 constitute a medical observation system. Note that, in the present first embodiment, the endoscope 2 and the imaging device 3 constitute an image acquisition device using an endoscope such as a rigid endoscope.

The light source device 6 includes two light source units (a first light source unit 61 and a second light source unit 62) each of which is connected to one end of a light guide 7, and a light source controller 63 that controls emission of illumination light by the light source units. In the present first embodiment, the first light source unit 61 supplies infrared (IR) light for illuminating inside a living body. The second light source unit 62 includes a light source that emits white light for illuminating inside the living body, and an illumination optical system that guides the white light emitted from the light source to the light guide 7. The configuration of the first light source unit 61 will be described later. Note that the light source device 6 and the control device 5 may be configured to be separated from each other and communicate with each other as illustrated in FIG. 1, or may be configured to be integrated with each other. Furthermore, in the present first embodiment, although an example will be described in which the first light source unit 61 emits the IR light, the light emitted by the first light source unit 61 is not limited to the IR light but light in a wavelength band for exciting the observation target is selected.

The light guide 7 has one end removably connected to the light source device 6 and the other end removably connected to the endoscope 2. Then the light guide 7 transmits, from the one end to the other end, the light supplied from the light source device 6 and supplies the light to the endoscope 2.

The imaging device 3 captures an object image transmitted from the endoscope 2 and outputs a result of the capturing image. As illustrated in FIG. 1, the imaging device 3 includes a transmission cable 8 and a camera head 9. In the present first embodiment, the transmission cable 8 and the camera head 9 constitute a medical imaging device.

The endoscope 2 is rigid and has an elongated shape to be inserted into a living body. On the inside of the endoscope 2, an observation optical system is provided that includes one or a plurality of lenses and focuses the object image. The endoscope 2 emits the light supplied via the light guide 7 from a distal end to irradiate the inside of the living body. Then, the light (the object image) emitted to the inside of the living body is focused by the observation optical system (lens unit 91) in the endoscope 2.

The camera head 9 is removably connected to a proximal end of the endoscope 2. Then, under the control of the control device 5, the camera head 9 captures the object image focused by the endoscope 2, and outputs an imaging signal obtained by the image capturing. Note that a detailed configuration of the camera head 9 will be described later. The endoscope 2 and the camera head 9 may be configured removably, as illustrated in FIG. 1, or may be integrated with each other.

The transmission cable 8 has one end removably connected to the control device 5 via a connector and the other end removably connected to the camera head 9 via a connector. Specifically, the transmission cable 8 is a cable in which a plurality of electric wires (not illustrated) is arranged inside an outer jacket as the outermost layer. Each of the plurality of electric wires supplies the imaging signal output from the camera head 9 to the control device 5, and supplies, to the camera head 9, a control signal, a synchronization signal, a clock, and power that are output from the control device 5.

Under the control of the control device 5, the display device 4 displays an image generated by the control device 5. In order to easily obtain immersive feeling during observation, the display device 4 preferably has, but is not limited to, a display unit of a size of 55 inches or more.

The control device 5 processes the imaging signal input from the camera head 9 via the transmission cable 8, outputs an image signal to the display device 4, and integrally controls the operations of the camera head 9 and the display device 4. Note that a detailed configuration of the control device 5 will be described later.

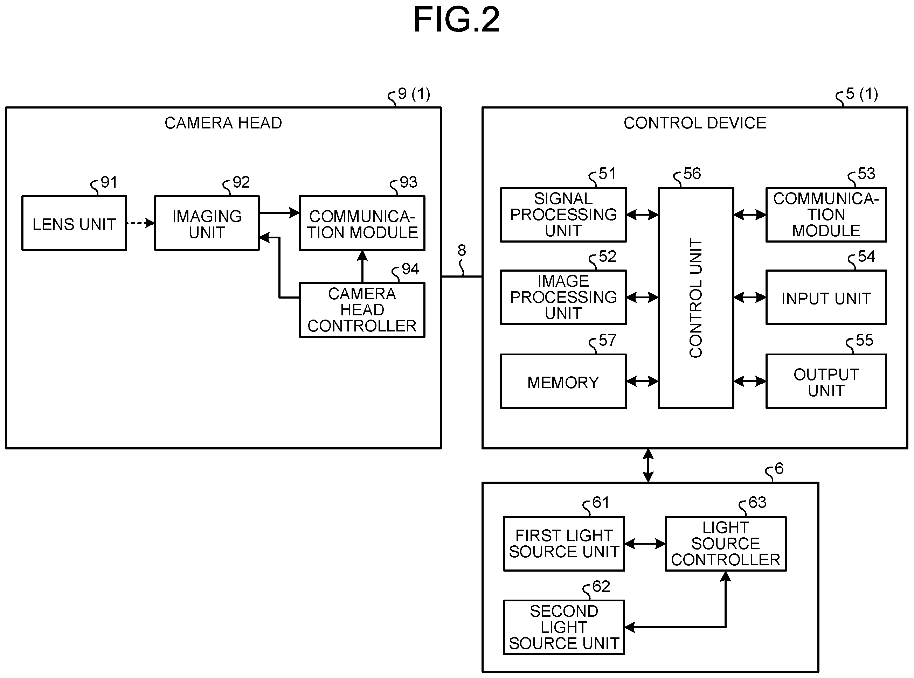

Next, the configurations of the imaging device 3 and the control device 5 will be described. FIG. 2 is a block diagram illustrating the configurations of the camera head 9, the control device 5, and the light source device 6. Note that in FIG. 2, illustration of the connector removably connecting the camera head 9 and the transmission cable 8 is omitted.

The configuration of the control device 5 and the configuration of the camera head 9 will be described below in this order. Note that in the following, as a configuration of the control device 5, a main portion of the present invention will mainly be described. As illustrated in FIG. 2, the control device 5 includes a signal processing unit 51, an image processing unit 52, a communication module 53, an input unit 54, an output unit 55, a control unit 56, and a memory 57. Note that in the control device 5, a power supply unit (not illustrated) or the like may be provided that generates power supply voltage for driving the control device 5 and the camera head 9, supplies the generated power supply voltage to each unit of the control device 5 and further to the camera head 9 via the transmission cable 8.

The signal processing unit 51 performs signal processing, such as noise removal or necessary A/D conversion, on the imaging signal output by the camera head 9, acquires a digitized imaging signal (pulse signal), and outputs the digitized imaging signal to the image processing unit 52.

Furthermore, the signal processing unit 51 generates the synchronization signal for the imaging device 3 and the control device 5, and the clock. The synchronization signal (e.g., the synchronization signal or the like for indicating imaging timing of the camera head 9) for the imaging device 3 and the clock (e.g., the clock for serial communication) are transmitted to the imaging device 3 via a line which is not illustrated, and the imaging device 3 is driven on the basis of the synchronization signal and the clock.

On the basis of the imaging signal input from the signal processing unit 51, the image processing unit 52 generates the image signal for display that is displayed by the display device 4. The image processing unit 52 performs predetermined signal processing on the imaging signal to generate the image signal for display that includes the object image. Here, the image processing unit 52 performs known image processing, for example, various types of image processing, such as detection processing, interpolation processing, color correction processing, color enhancement processing, and contour enhancement processing. The image processing unit 52 outputs the generated image signal to the display device 4.

The communication module 53 outputs signals from the control device 5, including the control signal, which is described later, transmitted from the control unit 56, to the imaging device 3. In addition, a signal from the imaging device 3 is output to the units in the control device 5. In other words, the communication module 53 is a relay device that collectively outputs signals output from the respective units of the control device 5, to the imaging device 3, for example, by parallel-to-serial conversion or the like, and delivers signals input from the imaging device 3, for example, by serial-to-parallel conversion or the like so as to be output to the respective units of the control device 5.

The input unit 54 is implemented by using a user interface such as a keyboard, mouse, or touch panel, and receives input of various types of information.

The output unit 55 is implemented by using a speaker, printer, or display and outputs various types of information. The output unit 55 outputs alarm sound or alarm light and displays an image, under the control of the control unit 56.

The control unit 56 performs drive control of each component unit including the control device 5 and the camera head 9, input/output control of information for each component unit, and the like. The control unit 56 refers to communication information data (e.g., communication format information, etc.) recorded in the memory 57 to generate the control signal, and transmits the generated control signal to the imaging device 3 via the communication module 53. Furthermore, the control unit 56 outputs the control signal to the camera head 9 via the transmission cable 8.

The memory 57 is implemented by using a semiconductor memory such as a flash memory or a dynamic random access memory (DRAM), and records the communication information data (e.g., communication format information, etc.). Note that various programs and the like executed by the control unit 56 may be recorded in the memory 57.

Note that the signal processing unit 51 may include an AF processing unit that outputs a predetermined AF evaluation value for each input frame on the basis of the imaging signal of the input frame, and an AF calculation unit that performs AF calculation to select a frame, focus lens position, or the like that is most suitable for a focus position, from the AF evaluation values of the respective frames output from the AF processing unit.

The signal processing unit 51, the image processing unit 52, the communication module 53, and the control unit 56, which are described above, are implemented by using a general-purpose processor, such as a central processing unit (CPU), having an internal memory (not illustrated) in which a program is recorded, and a dedicated processor for various arithmetic circuits or the like such as an application specific integrated circuit (ASIC) that performs a specific function. Furthermore, the signal processing unit 51, the image processing unit 52, the communication module 53, and the control unit 56 may include a field programmable gate array (FPGA: not illustrated) that is a type of programmable integrated circuit. Note that when the FPGA is included, a memory that stores configuration data may be provided so that the FPGA as the programmable integrated circuit is configured on the basis of the configuration data read from the memory.

Next, as a configuration of the camera head 9, a main portion of the present invention will be described mainly. As illustrated in FIG. 2, the camera head 9 includes the lens unit 91, an imaging unit 92, a communication module 93, and a camera head controller 94.

The lens unit 91 includes one or a plurality of lenses, and forms the object image passing through the lens unit 91, on an imaging surface of an image sensor constituting the imaging unit 92. The one or a plurality of lenses is configured to be movable along the optical axis. Then, the lens unit 91 is provided with an optical zoom mechanism (not illustrated) that moves the one or a plurality of lenses to change the angle of view, and a focus mechanism that changes the focal position. Note that the lens unit 91 forms the observation optical system that guides observation light entering the endoscope 2 to the imaging unit 92, together with the optical system provided in the endoscope 2.

Under the control by the camera head controller 94, the imaging unit 92 images the object. The imaging unit 92 includes the image sensor that receives the object image formed by the lens unit 91 and converts the object image into an electric signal. The image sensor includes a charge coupled device (CCD) image sensor or a complementary metal oxide semiconductor (CMOS) image sensor. In a case where the image sensor includes the CCD, for example, a signal processing unit (not illustrated) that performs signal processing (A/D conversion, etc.) on an electric signal (analog signal) from the image sensor to output the imaging signal is mounted on a sensor chip or the like. In a case where the image sensor includes the CMOS, for example, a signal processing unit (not illustrated) that performs the signal processing (A/D conversion, etc.) on the electric signal (analog signal) obtained by converting light and outputs the imaging signal is included in the image sensor. The imaging unit 92 outputs the generated electric signal to the communication module 93.

The communication module 93 outputs the signal transmitted from the control device 5 to each unit in the camera head 9 such as the camera head controller 94. Furthermore, the communication module 93 converts information or the like about the current state of the camera head 9 into a signal format according to a predetermined transmission method, and outputs the signal after the conversion to the control device 5 via the transmission cable 8. In other words, the communication module 93 is a relay device that delivers signals input from the control device 5 and the transmission cable 8, for example, by the serial-to-parallel conversion or the like and outputs the signals to the respective units of the camera head 9, and collectively outputs signals from the respective units of the camera head 9, to the control device 5 and the transmission cable 8, for example, by the parallel-to-serial conversion or the like.

The camera head controller 94 controls the entire operation of the camera head 9 according to a drive signal input via the transmission cable 8, an instruction signal output from an operating unit such as a switch provided to be exposed from an outer surface of the camera head 9 by the user's operation to the operating unit, or the like. In addition, the camera head controller 94 outputs the information about the current state of the camera head 9 to the control device 5 via the transmission cable 8.

Note that the communication module 93 and the camera head controller 94, which are described above, are implemented by using a general-purpose processor, such as a CPU, having an internal memory (not illustrated) in which a program is recorded, and a dedicated processor for various arithmetic circuits or the like, such as ASIC, that performs a specific function. Furthermore, the communication module 93 and the camera head controller 94 may include an FPGA that is a type of programmable integrated circuit. Here when the FPGA is included, a memory that stores configuration data may be provided so that the FPGA as the programmable integrated circuit is configured on the basis of the configuration data read from the memory.

Note that the camera head 9 or the transmission cable 8 may include a signal processing unit that performs signal processing on the imaging signal generated by the communication module 93 or the imaging unit 92. Furthermore, on the basis of a reference clock generated by an oscillator (not illustrated) provided in the camera head 9, an imaging clock for driving the imaging unit 92 and a controlling clock for the camera head controller 94 may be generated to be output to the imaging unit 92 and the camera head controller 94, or on the basis of the synchronization signal input from the control device 5 via the transmission cable 8, timing signals for various types of processing in the imaging unit 92, the camera head controller 94 may be generated to be output to the imaging unit 92 and the camera head controller 94. Furthermore, the camera head controller 94 may be provided in the transmission cable 8 or the control device 5 instead of at the camera head 9.

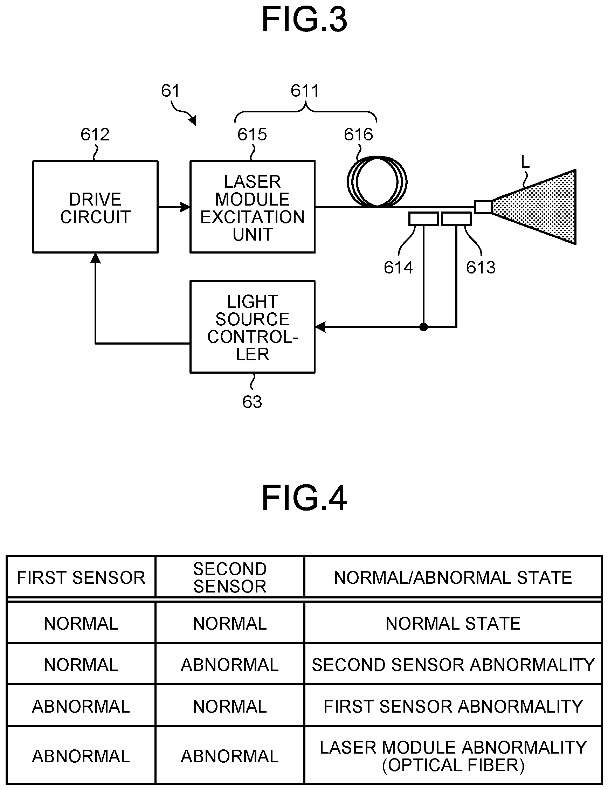

Next, a configuration of a main portion of the light source device 6 will be described with reference to FIG. 3. FIG. 3 is a diagram illustrating a configuration of a main portion of the light source device illustrated in FIG. 1. The first light source unit 61 includes a laser module 611 that emits infrared light, a drive circuit 612 that drives the laser module 611, and two detection units (a first sensor 613 and a second sensor 614) that detect light guided by the laser module. These units are provided inside a casing constituting the light source device 6. Note that the medical lighting device according to the present invention is constituted by at least the laser module 611, the two detection units (the first sensor 613 and the second sensor 614), and the light source controller 63.

The laser module 611 includes a laser module excitation unit 615 that emits laser light (infrared light) and an optical fiber 616 that guides the laser light emitted by the laser module excitation unit 615.

The laser module excitation unit 615 excites a light emitter with current supplied from the drive circuit 612 and emits the laser light. The laser light emitted from the laser module excitation unit 615 is guided to one end of the optical fiber 616 and emitted from the other end of the optical fiber 616. The laser light emitted from the other end of the optical fiber 616 enters the light guide 7 through a filter and an optical system, which are not illustrated. The laser module excitation unit 615 corresponds to an emission unit.

The optical fiber 616 is bent according to an arrangement space in the casing of the light source device 6. For example, the optical fiber 616 is partially wound as illustrated in FIG. 3. The optical fiber 616 includes a single fiber. Note that the optical fiber 616 may include a bundle of a plurality of fibers.

The first sensor 613 and the second sensor 614 each include an optical sensor, and are provided on a side surface of the optical fiber 616 and in the vicinity of an emission end of the optical fiber 616. The first sensor 613 and the second sensor 614 each receive light leaking from the side surface of the optical fiber 616 and output a light amount value according to an amount of light received. The first sensor 613 and the second sensor 614 may be arranged side by side in a longitudinal direction of the optical fiber 616 as illustrated in FIG. 3, or may be arranged side by side in a circumferential direction of the optical fiber 616.

In response to reception of detection values from the first sensor 613 and the second sensor 614, the light source controller 63 determines normality/abnormality in the detection values of the first sensor 613 and the second sensor 614, on the basis of the detection values and a preset reference value. For example, the light source controller 63 calculates a difference between each of the detection values and the reference value, and determines the normality/abnormality according to the difference. The reference value is associated with, for example, an amount of light emitted by the laser module excitation unit 615, and, for the reference value, a theoretical detection value is set that is to be detected in guiding light of an amount corresponding to the amount of light emitted, by the optical fiber 616. Furthermore, the light source controller 63 stores a determination threshold for determining normality/abnormality on the basis of the calculated difference.

The light source controller 63 is implemented by using a general-purpose processor, such as a CPU, having an internal memory (not illustrated) in which a program is recorded, a dedicated processor for various arithmetic circuits or the like, such as ASIC, that performs a specific function, and a memory.

After determining the normality/abnormality in the first sensor 613 and the second sensor 614, the light source controller 63 determines the normal/abnormal state of each of the first sensor 613, the second sensor 614, and the laser module 611, on the basis of a combination of results of the determination.

FIG. 4 is a diagram illustrating determination patterns for determining the normal/abnormal state in a light source unit, by the light source controller. When determining that both of the detection values of the first sensor 613 and the second sensor 614 are normal, the light source controller 63 determines that the first sensor 613, the second sensor 614, and the laser module 611 are in the normal state.

On the other hand, when determining that one of the first sensor 613 and the second sensor 614 is normal and the other is abnormal, the light source controller 63 determines that either sensor determined to be abnormal is in the abnormal state. Specifically, when determining that the first sensor is normal and the second sensor 614 is abnormal, the light source controller 63 determines that the second sensor is in the abnormal state. On the other hand, when determining that the first sensor is abnormal and the second sensor 614 is normal, the light source controller 63 determines that the first sensor is in the abnormal state.

Furthermore, when determining that both the first sensor and the second sensor 614 are abnormal, the light source controller 63 determines that the optical fiber 616 is abnormal. At this time, in the optical fiber 616, light is leaked to the outside before the light reaches the emission end, for example, due to fiber breakage or the like.

The light source controller 63 outputs information about a result of the determination to the control device 5. The light source controller 63 corresponds to a control unit of the medical lighting device. Note that, separately from the light source controller 63, a control unit that determines the normality/abnormality and outputs the information about a result of the determination to the outside may be provided, or a control unit that includes a determination unit determining the normality/abnormality and an output unit outputting the information about the result of the determination to the outside may be provided.

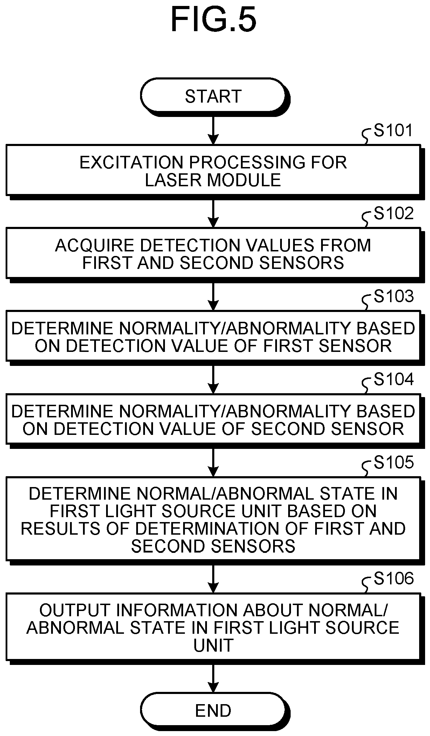

Next, a process of determining the normal/abnormal state in the first light source unit 61 by the light source controller 63 will be described with reference to FIG. 5. FIG. 5 is a flowchart illustrating the process of determining the normal/abnormal state in the first light source unit by the light source controller according to the first embodiment of the present invention.

The light source controller 63 performs excitation processing for the laser module 611 (Step S101). Therefore, laser light is emitted from the laser module excitation unit 615. The laser light emitted from the laser module excitation unit 615 enters the optical fiber 616, travels in the fiber, and is emitted to the outside.

After the excitation processing for the laser module 611, the light source controller 63 acquires the detection values from the first sensor 613 and the second sensor 614 (Step S102).

In response to reception of the detection value from the first sensor 613, the light source controller 63 determines the normality/abnormality in the detection value of the first sensor 613, on the basis of the detection value and the preset reference value (Step S103). The light source controller 63 calculates a difference between the detection value and the reference value, and determines the normality/abnormality in the detection value of the first sensor 613 according to the difference.

In response to reception of the detection value from the second sensor 614, the light source controller 63 determines the normality/abnormality in the detection value of the second sensor 614, on the basis of the detection value and the preset reference value (Step S104). The light source controller 63 calculates a difference between the detection value and the reference value, and determines the normality/abnormality in the detection value of the second sensor 614 according to the difference.

Note that Step S103 and Step S104 may be reversed or may be performed simultaneously.

After determining the states of the first sensor 613 and the second sensor 614, on the basis of results of the determination, the light source controller 63 determines the normal/abnormal state in the first light source unit 61 (Step S105). On the basis of a combination of the result of the determination of the first sensor 613 and the result of the determination of the second sensor 614, the light source controller 63 determines the normal/abnormal state in the first light source unit 61 (e.g., see FIG. 4).

After determination of the normal/abnormal state in the first light source unit 61, the light source controller 63 outputs the information about a result of the determination (Step S106). The light source controller 63 outputs information about a result of the determination to the control device 5. The control device 5 processes the result of the determination, on the basis of a preset condition. For example, the control device 5 causes the output unit 55 to output the result of the determination. The output unit 55 displays the result of the determination, for example, on the monitor. The output unit 55 displays, as the result of the determination, any of normal, first sensor abnormality, second sensor abnormality, and optical fiber abnormality. Note that the output unit 55 may display the results of the determination of the normality/abnormality in the detection values of the first sensor 613 and the second sensor 614. In this configuration, an operator or the like checks the results of the determination in the detection values of the first sensor 613 and the second sensor 614, and determines any of the first sensor abnormality, the second sensor abnormality, and the optical fiber abnormality. Furthermore, the results of the determination of the states in the first sensor 613 and the second sensor 614 may be transmitted to a terminal of a person in charge of maintenance of the device.

In addition, the light source controller 63 controls the laser module excitation unit 615 on the basis of the result of the determination of the normal/abnormal state in the first light source unit 61. For example, in a case where it is determined that any of the first sensor 613, the second sensor 614, and the optical fiber 616 has an abnormality, the light source controller 63 may stop the emission of the laser light by the laser module excitation unit 615. Furthermore, in a case where it is determined that only one of the first sensor 613 and the second sensor 614 has an abnormality, the light source controller 63 may continue the emission of the laser light by the laser module excitation unit 615. After continuation of emission of the laser light, only one sensor that is normal is used for the detection. Furthermore, when one of the sensors performing the detection is detected to be abnormal, emission of the laser light may be stopped and the abnormality of the one sensor may be displayed (notified of) on the display device 4 as error information.

In the first embodiment described above, the two sensors (the first sensor 613 and the second sensor 614) are provided on the emission end side of the optical fiber 616, and each state of the normal, the first sensor abnormality, the second sensor abnormality, and the optical fiber abnormality is determined on the basis of the results of the determination of the normality/abnormality in the detection values of the respective sensors. Therefore, it is possible to identify which of the optical fiber and the optical sensors has an abnormal portion. According to the present first embodiment, the abnormal portion is identified, and thus, it is not necessary to take out and check all of the laser module 611, the first sensor 613 and the second sensor 614, and it is only required to take out and replace a component having the abnormal portion to eliminate the abnormal state. At this time, the first sensor 613 and the second sensor 614 are provided in the vicinity of the emission end of the optical fiber 616 and have substantially the same detection values, and thus an identical determination value can be used.

In the above-described first embodiment, the optical sensor described above is provided in the first light source unit 61 that emits the IR light, and the normality/abnormality of the sensors and the optical fiber is detected. Here, the IR light is invisible to the human eye, and it is difficult to know that the light is actually emitted or the emitted light has how much intensity. In the first embodiment, such an emission state of light that cannot be seen with the human eye and cannot be judged by the human eye is determined by using the detection values of the optical sensors.

Note that in the first embodiment described above, the example has been described in which the first sensor 613 and the second sensor 614 that are provided in the vicinity of the emission end of the optical fiber 616. However, in order to determine abnormality such as breakage of the entire optical fiber 616, the first sensor 613 and the second sensor 614 are preferably provided at positions closer to the emission end of the optical fiber 616. In other words, the first sensor 613 and the second sensor 614 are preferably provided in the vicinity of the emission end, including the emission end for the light of the optical fiber 616.

### (Second Embodiment)

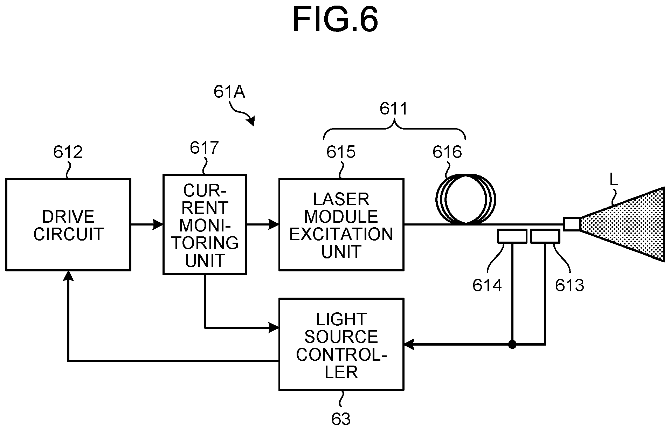

Next, a second embodiment of the present invention will be described. FIG. 6 is a diagram illustrating a configuration of a main portion of a light source device in an endoscope apparatus according to a second embodiment of the present invention. In the present second embodiment, a first light source unit 61A is included instead of the first light source unit 61 of the first embodiment described above. In the present second embodiment, the configuration other than the configuration of the first light source unit 61A is the same as that of the endoscope apparatus 1 of the first embodiment described above, and thus the description thereof will be omitted.

The first light source unit 61A includes the laser module 611 that emits infrared light, the drive circuit 612 that drives the laser module 611, and two detection units (the first sensor 613 and the second sensor 614) that detect light guided by the laser module, and a current monitoring unit 617. These units are provided inside the casing constituting the light source device 6. The configurations of the laser module 611, the drive circuit 612, and the detection units are similar to those in the first embodiment, and thus the description thereof is omitted.

The current monitoring unit 617 monitors current supplied from the drive circuit 612 to the laser module excitation unit 615. Specifically, the current monitoring unit 617 detects the magnitude (current value) of current flowing between the drive circuit 612 and the laser module excitation unit 615. The current monitoring unit 617 outputs the detected current value (detection value) to the light source controller 63.

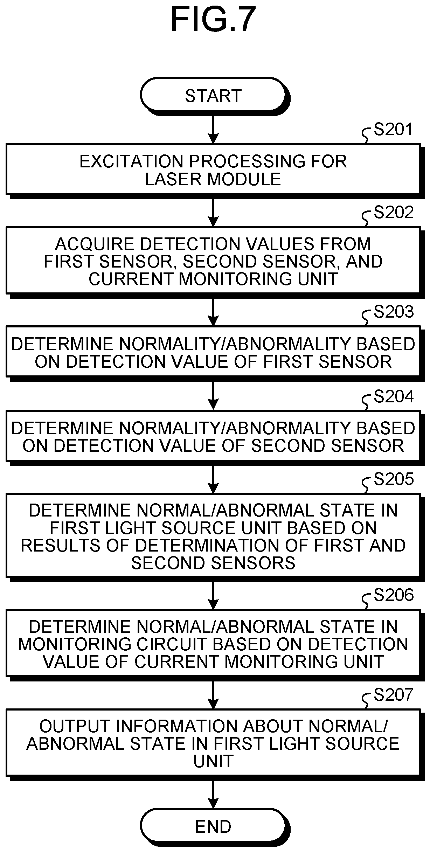

Next, a process of determining the normal/abnormal state in the first light source unit 61A by the light source controller 63 will be described with reference to FIG. 7. FIG. 7 is a flowchart illustrating a process of determining the normal/abnormal state in the first light source unit by the light source controller according to the second embodiment of the present invention.

The light source controller 63 performs excitation processing for the laser module 611 (Step S201). Laser light emitted from the laser module excitation unit 615 enters the optical fiber 616, travels in the fiber, and is emitted to the outside.

After the excitation processing for the laser module 611, the light source controller 63 acquires the detection values from the first sensor 613, the second sensor 614, and the current monitoring unit 617 (Step S202).

In response to reception of the detection value from the first sensor 613, the light source controller 63 determines the normality/abnormality in the detection value of the first sensor 613, on the basis of the detection value and a preset reference value (Step S203).

In response to reception of the detection value from the second sensor 614, the light source controller 63 determines the normality/abnormality in the detection value of the second sensor 614, on the basis of the detection value and the preset reference value (Step S204).

Note that Steps S203 and S204 may be reversed or may be performed simultaneously.

After determining the states of the first sensor 613 and the second sensor 614, on the basis of results of the determination, the light source controller 63 determines the normal/abnormal state in the first light source unit 61A (Step S205). On the basis of a combination of the result of the determination of the first sensor 613 and the result of the determination of the second sensor 614, the light source controller 63 determines the normal/abnormal state in the first light source unit 61A (e.g., see FIG. 4).

The light source controller 63 determines the normal/abnormal state in the current monitoring unit 617 on the basis of the detection value acquired from the current monitoring unit 617 (Step S206). As in Step S105 described above, the light source controller 63 determines the normality/abnormality in the detection value of the current monitoring unit 617, on the basis of the detection value and the preset reference value.

After determination of the normal/abnormal state in the first light source unit 61A (detection values of the first sensor 613, the second sensor 614, and the current monitoring unit 617), the light source controller 63 outputs information about a result of the determination (Step S207). The light source controller 63 outputs information about a result of the determination to the control device 5. The control device 5 processes the result of the determination, on the basis of a preset condition. For example, the control device 5 causes the output unit 55 to output the result of the determination. The output unit 55 displays the result of the determination, for example, on the monitor. The output unit 55 displays the result of the determination that is selected from the normal, first sensor abnormality, second sensor abnormality, and optical fiber abnormality on the basis of the result of the determination.

In the second embodiment described above, the two sensors (the first sensor 613 and the second sensor 614) are provided on the emission end side of the optical fiber 616, and each state of the normal, the first sensor abnormality, the second sensor abnormality, and the optical fiber abnormality is determined on the basis of the results of the determination of the normality/abnormality in the detection values of the respective sensors. Therefore, it is possible to identify which of the optical fiber and the optical sensors has an abnormal portion. Furthermore, in the present second embodiment, the current monitoring unit 617 is provided between the drive circuit 612 and the laser module excitation unit 615 so as to determine the abnormality of a portion for monitoring the current value of current flowing between the drive circuit 612 and the laser module excitation unit 615. According to the present second embodiment, the abnormal portion is identified, and thus, it is not necessary to take out and check all of the laser module 611, the first sensor 613 and the second sensor 614, and the current monitoring unit 617, and it is only required to take out and replace a component having the abnormal portion to eliminate the abnormal state.

Although the modes for carrying out the present invention have been described, the present invention should not be limited only by the embodiments described above. In the description of the above embodiments, the control device 5 performs signal processing and the like, but the camera head 9 may perform the signal processing and the like.

Note that in the first and second embodiments described above, an optical sensor may also be provided on the light entrance end side of the optical fiber 616 to detect light transmitted between the laser module excitation unit 615 and the optical fiber 616.

Furthermore, in the first and second embodiments described above, the example has been described in which the normality/abnormality of the optical sensor and the optical fiber is determined for the light source that emits excitation light. However, the configuration for the determination described above may be applied to the light source that emits white light (e.g., the second light source unit 62 described above).

Still furthermore, the first and second embodiments described above may have a configuration in which two detection units are arranged at an end portion of the light guide 7 connected to the endoscope 2 so as to determine the normality/abnormality of a light guiding path in the light guide 7.

Still another furthermore, in the first and second embodiments described above, the example has been described in which the light source controller 63 outputs, as the result of the determination, any of the normal, first sensor abnormality, second sensor abnormality, and optical fiber abnormality, on the basis of the detection values. However, only the normality/abnormality in the detection values of the first sensor and the second sensor may be output. In this case, for example, the results of the determination in Steps S103 and S104 of FIG. 5 described above are output.

In addition, execution programs for processes performed by the light source device according to the present first and second embodiments and another component unit may be configured to be provided by being recorded in a computer-readable recording medium such as a CD-ROM, flexible disk, CD-R, or DVD, in an installable or executable file format, or may be configured to be provided by being stored on a computer connected to a network such as the Internet and downloaded via the network. Furthermore, the execution programs may be provided or distributed via the network such as the Internet.

### (Another Embodiment)

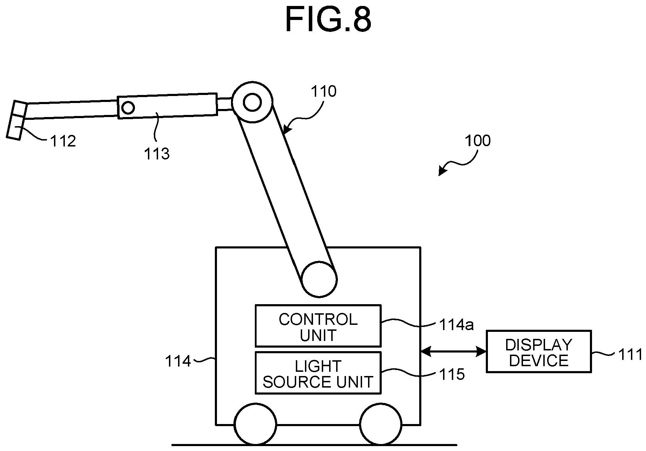

Furthermore, in the first and second embodiments described above, the example of the rigid endoscope has been described, but the present invention may be applied to a flexible endoscope, and can also be applied to a surgical microscope system (medical image acquisition system) having a function of enlarging and capturing an image of a predetermined field of view and displaying the captured image. FIG. 8 is a diagram schematically illustrating an overall configuration of the surgical microscope system as the medical observation system including a medical observation device according to another embodiment.

A surgical microscope system 100 includes a microscope device 110 as the medical imaging device that captures and acquires an image for observing the object, and a display device 111 that displays the image captured by the microscope device 110. Note that the display device 111 can also be configured integrally with the microscope device 110.

The microscope device 110 includes a microscope unit 112 that enlarges and captures a minute portion of the object, a support unit 113 that includes an arm connected to a proximal end portion of the microscope unit 112 and turnably supporting the microscope unit 112, and a base portion 114 that turnably holds the proximal end portion of the support unit 113 and is movable on a floor surface. The base portion 114 includes a control unit 114a that controls the operation of the surgical microscope system 100, and a light source unit 115 that generates illumination light to be emitted from the microscope device 110 to the object. Note that the control unit 114a has the function of the control device 5 described above, such as the signal processing unit 51 and the image processing unit 52 which are described above. Furthermore, the base portion 114 may be configured to be fixed to a ceiling, a wall surface, or the like to support the support unit 113, instead of being provided to be movable on the floor surface.

For example, the microscope unit 112 has a column shape and includes therein the imaging unit 92 described above. A switch that receives an input of an operation instruction for the microscope device 110 is provided on a side surface of the microscope unit 112. A cover glass that protects the inside is provided at an opening surface positioned at a lower end of the microscope unit 112 (not illustrated).

The light source unit 115 has the same configuration as that of the light source device of any of the first and second embodiments described above.

The user such as the operator holding the microscope unit 112 moves the microscope unit 112, performs a zoom operation, or switches illumination light while operating various switches. Note that the microscope unit 112 preferably has a shape elongated in an observation direction so that the user can easily hold and change the viewing direction. Therefore, the microscope unit 112 may have a shape other than the column shape, and may have, for example, a polygonal column shape.

In the control unit 114a, as in the first embodiment and the like, the light source controller 63 determines the normal/abnormal states of the first sensor 613, the second sensor 614, and the laser module 611, on the basis of the detection values of the first sensor 613 and the second sensor 614.

As described above, also in the surgical microscope system 100, the same effects as those of the first embodiment described above can be obtained.

### Industrial Applicability

As described above, the medical lighting device according to the present invention is useful to identify an abnormal portion in the optical fiber and the optical sensor.

### Reference Signs List

- 1: ENDOSCOPE APPARATUS
- 2: ENDOSCOPE
- 3: IMAGING DEVICE
- 4: DISPLAY DEVICE
- 5: CONTROL DEVICE
- 6: LIGHT SOURCE DEVICE
- 7: LIGHT GUIDE
- 8: TRANSMISSION CABLE
- 9: CAMERA HEAD
- 51: SIGNAL PROCESSING UNIT
- 52: IMAGE PROCESSING UNIT
- 53: COMMUNICATION MODULE
- 54: INPUT UNIT
- 55: OUTPUT UNIT
- 56,: 114a CONTROL UNIT
- 57: MEMORY
- 61, 61A: FIRST LIGHT SOURCE UNIT
- 62: SECOND LIGHT SOURCE UNIT
- 63: LIGHT SOURCE CONTROLLER
- 91: LENS UNIT
- 92: IMAGING UNIT
- 93: COMMUNICATION MODULE
- 94: CAMERA HEAD CONTROLLER
- 100: SURGICAL MICROSCOPE SYSTEM
- 110: MICROSCOPE DEVICE
- 111: DISPLAY DEVICE
- 112: MICROSCOPE UNIT
- 113: SUPPORT UNIT
- 114: BASE PORTION
- 115: LIGHT SOURCE UNIT

## Claims

1. A medical lighting device comprising:
an emission unit configured to emit light;
an optical fiber configured to guide and emit the light emitted from the emission unit and entered into the optical fiber;
first and second detection units provided in a vicinity of an emission end including an emission end for the light of the optical fiber and configured to detect the light in the optical fiber; and
a control unit configured to determine, based on detection values of the light detected by the first and second detection units, normality/abnormality in the detection values of the respective detection units and outputs determined results.

2. The medical lighting device according to claim 1, wherein
the control unit is configured to determine, based on results of the determination of the normality/abnormality in the detection values of the respective detection units, normality/abnormality of the first and second detection units and the optical fiber.

3. The medical lighting device according to claim 2, wherein
the control unit is configured to control to stop emission of the light by the emission unit in a case where at least one of the first and second detection units and the optical fiber is determined to be abnormal.

4. The medical lighting device according to claim 2, wherein
the control unit is configured to determine that the second detection unit is abnormal in a case where the detection value of the first detection unit is normal and the detection value of the second detection unit is abnormal.

5. The medical lighting device according to claim 2, wherein
the control unit is configured to determine that the optical fiber is abnormal in a case where the detection value of the first detection unit and the detection value of the second detection unit are abnormal.

6. The medical lighting device according to claim 1, wherein
the light emitted from the emission unit is excitation light for exciting an irradiation target.

7. The medical lighting device according to claim 1, further comprising
a current monitoring unit configured to monitor current supplied to the emission unit,
wherein the control unit is configured to
determine normality/abnormality of the detection unit, and
determine normality/abnormality of the current monitoring unit.

8. The medical lighting device according to claim 1, wherein
the optical fiber includes a single fiber.
